# EUROPÄISCHE PATENTANMELDUNG

(11) **EP 4 763 044 A1**
(43) Veröffentlichungstag der Anmeldung: **24.06.2026**
(21) Anmeldenummer: 25223587.4
(22) Anmeldetag: 15.12.2025
(51) Int. Cl.: A61B 1/00, A61B 1/06, A61B 1/07, A61B 1/005, A61B 1/05, A61B 1/12

(54) **ENDOSKOPVORRICHTUNG UND ENDOSKOP MIT EINER ENDOSKOPVORRICHTUNG**

(30) Priorität: 19.12.2024 DE 102024138870
(71) Anmelder: Karl Storz SE & Co. KG, 78532 Tuttlingen (DE)
(72) Erfinder: SCHWARZ, Peter, 78532 Tuttlingen (DE)

(57) **Zusammenfassung**

Die Erfindung betrifft eine Endoskopvorrichtung (10), umfassend: einen Schaft (20) mit einer Schaftlängsachse (30); eine Kameraaufnahme (40); wenigstens ein Gliederelement (50), an dem die Kameraaufnahme (40) beweglich befestigt ist; ein Kameramodul (60), das eine stereoskopische Bilderfassungseinheit (70) aufweist und in der Kameraaufnahme (40) aufnehmbar ist, wobei eine Bewegbarkeit der Kameraaufnahme (40) relativ zu dem Gliederelement (50) durch ein Verschieben der Kameraaufnahme (40) und des Gliederelements (50) relativ zu dem Schaft (20) freigebbar ist, wodurch das Kameramodul (60) von einer Einführlage in eine Bildaufnahmelage auslenkbar ist, wobei in der Bildaufnahmelage das Kameramodul (60) eine Blickrichtung definiert, die zu der Schaftlängsachse winklig ist, und wobei in der Bildaufnahmelage die Bilderfassungseinheit (70) zu einer Bildaufrichtung relativ zu der Kameraaufnahme (40) drehbar gelagert ist.

Weiter betrifft die Erfindung ein Endoskop mit einer erfindungsgemäßen Endoskopvorrichtung.

## Beschreibung

Die vorliegende Erfindung betrifft eine Endoskopvorrichtung, insbesondere ein Stereo-Laparoskop, und ein Endoskop mit einer Endoskopvorrichtung.

In der minimalinvasiven Chirurgie gehören Endoskope zum Stand der Technik, mittels derer Vergrößerungsdarstellungen eines Untersuchungsgebiets innerhalb eines Körpers eines Patienten erzeugt werden können. Zur besseren Visualisierung werden in Endoskopen stereoskopische Bilderfassungseinheiten eingesetzt, die die Umgebung einem Operateur und/oder einem weiteren Benutzer, in 3D und/oder 2D visualisieren können.

Um dem Operateur und/oder dem weiteren Benutzer einen guten Überblick zu gewähren, sind die Bilderfassungseinheiten meist zu einer Längsachse des Endoskops hin abgewinkelt, um Bilder winklig zur Längsachse des Endoskops und damit einen sogenannten Schrägblick visualisieren zu können. Dazu werden meistens Endoskope eingesetzt, die einen 30° und/oder 45° und/oder 60° und/oder 75° Schrägblick aufweisen.

Bei der Realisierung von Endoskopen mit stereoskopischer Bilderfassungseinheit, die im Einsatz einen Schrägblick aufweisen sollen, ist es besonders schwierig, insbesondere die Außendurchmesser derart zu reduzieren, dass der minimalinvasive Eingriff so gering wie möglich gehalten werden kann. Weiter soll zur besseren Orientierung des Anwenders bei der 3D- und/oder 2D-Darstellung ggf. das Bild der Bilderfassungseinheit aufrichtbar sein. Während 2D-Darstellungen auch elektronisch aufgerichtet werden können, ist dies bei 3D-Darstellungen nicht möglich. Hier kann eine Bildaufrichtung zum Beispiel durch aufwändige mechanische Systeme zur Bewegung der einzelnen Bildaufnehmer realisiert werden.

Weiter gilt es am distalen Ende des Endoskops ausreichend Licht zur Beleuchtung bereitzustellen, um das Innere des Körpers des Patienten ausreichend ausleuchten zu können, um bestmögliche Bilder aufzeichnen zu können.

Zusätzlich können durch die medizinische Bildgebung der Endoskope unterschiedliche Gewebearten, beispielsweise Organe und/oder Blutgefäße und/oder Tumorgewebe, in unterschiedlichen Tiefen unter der Haut oder in der Körperhöhle visualisiert werden. Um die Gewebearten besser differenzieren zu können, werden Fluoreszenzbildgebungsverfahren eingesetzt. Dazu wird dem Patienten ein Medikament mit einem Fluoreszenzfarbstoff, insbesondere Fluorophoren, verabreicht, das sich in einer der unterschiedlichen Gewebearten ablagert. Insbesondere bei diesen Fluoreszenzbildgebungsverfahren gilt es am distalen Ende des Endoskops ausreichend Anregungslicht zur Verfügung zu stellen, um die fluoreszierenden Substanzen ausreichend anregen zu können.

In bekannten Endoskopen mit Schrägblick werden distale Prismen eingesetzt, um den Schrägblick zu realisieren. Besonders bei Endoskopen mit Schrägblick stellt die Integration von ausreichend Beleuchtungsfasern eine hohe Herausforderung dar, da durch die distalen Prismen der Raum innerhalb des Endoskops zur Integration von Beleuchtungsfasern nur sehr eingeschränkt vorhanden ist.

Überdies ist es wichtig, das distale Ende des Endoskops während des Einsatzes spülen zu können. Zur Spülung des distalen Endes des Endoskops wird zumeist ein Fluid eingesetzt, das über entsprechende Leitungen an das distale Ende des Endoskops geführt wird. Allerdings wird für die Integration der Leitungen in bekannten Endoskopen teilweise ein zusätzlicher Spülschaft benötigt, der zusätzlich auf das Endoskop gesteckt werden muss. Durch den zusätzlichen Schaft wird der Außendurchmesser des Endoskops unnötig vergrößert. Daher muss aufgrund des zusätzlichen Schafts ein Trokar mit einem größeren Durchmesser eingesetzt werden, der den minimalinvasiven Eingriff unnötig vergrößert.

Die DE10004264C2 zeigt eine Stereokamera, die über Gelenke vom Schaft eines Endoskops abgespreizt werden kann. Durch den Vorschub eines Instruments wird die Abspreizung gesteuert und das Instrument im Blickfeld der Kamera gehalten. WO2014104405A1 beschreibt ein Endoskop mit einer abwinkelbaren Stereokamera, die mit einem Schubmechanismus in die abgewinkelte Position gebracht werden kann.

Ein ähnlicher Mechanismus ist auch aus der EP2123225A1 bekannt.

In der US11064867B2 ist ein Endoskop offenbart, wobei eine distale Kameraeinheit durch Vorschub eines Hohlschaftes innerhalb des Instruments nach außen abgelenkt und so in eine seitlich zum Schaft versetzte Position verfahren wird.

Ausgehend vom Stand der Technik liegt der Erfindung insbesondere, aber nicht beschränkt darauf, die Aufgabe zugrunde, ein Endoskop mit Schrägblick zu schaffen, das einem Benutzer eine intuitive Bildaufrichtung ermöglicht.

Weiter liegt der Erfindung ausgehend vom Stand der Technik insbesondere, aber nicht beschränkt darauf, die Aufgabe zugrunde, mit einem möglichst kleinen minimalinvasiven Eingriff viel Raum, beispielsweise für die Integration von Beleuchtungsfasern und/oder Fluidleitungen, zur Verfügung zu stellen.

Wenigstens eine dieser Aufgaben wird erfindungsgemäß durch eine Endoskopvorrichtung und ein Endoskop mit einer Endoskopvorrichtung gelöst, wie sie hierin beschrieben und in den Ansprüchen definiert sind.

Die vorliegende Erfindung sieht dazu eine Endoskopvorrichtung mit einem Schaft mit einer Schaftlängsachse vor. Die Endoskopvorrichtung umfasst weiter eine Kameraaufnahme, wenigstens ein Gliederelement, an dem die Kameraaufnahme beweglich befestigt ist und ein Kameramodul, das eine stereoskopische Bilderfassungseinheit aufweist und in der Kameraaufnahme aufnehmbar ist. Weiter ist in der erfindungsgemäßen Endoskopvorrichtung eine, insbesondere gelenkige, Bewegbarkeit der Kameraaufnahme relativ zu dem Gliederelement durch ein Verschieben der Kameraaufnahme und des Gliederelements relativ zu dem Schaft freigebbar, wodurch das Kameramodul von einer Einführlage in eine Bildaufnahmelage auslenkbar ist. Weiter definiert das Kameramodul in der Bildaufnahmelage eine Blickrichtung, die zu der Schaftlängsachse winklig ist. Zusätzlich ist in der Bildaufnahmelage die Bilderfassungseinheit zu einer Bildaufrichtung relativ zu der Kameraaufnahmedrehbar gelagert.

Die durch die Freigabe der Bewegbarkeit ermöglichte Bewegung ist oder umfasst insbesondere eine Auslenkung.

Insbesondere kann das Kameramodul und/oder die Kameraaufnahme und/oder das wenigstens eine Gliederelement in einer Einführlage in den Schaft aufgenommen sein. Insbesondere wird die Endoskopvorrichtung in der Einführlage in das Körperinnere des Patienten eingeführt. Beispielweise kann die Endoskopvorrichtung durch einen Trokar in das Körperinnere eingeführt werden. In der Bildaufnahmelage weist die Endoskopvorrichtung insbesondere einen Schrägblick auf. Der Schrägblick kann beispielsweise 30° oder 45° oder 60° oder 75° betragen. Beispielsweise kann die Endoskopvorrichtung in der Bildaufnahmelage dazu eingerichtet sein, ein Bild einer Umgebung, beispielsweise von dem Inneren des Körpers des Patienten, aufzuzeichnen.

Insbesondere erstreckt sich der Schaft von einem proximalen Ende zu einem distalen Ende der Endoskopvorrichtung. Insbesondere ist der Schaft starr und/oder flexibel ausgebildet. Insbesondere ist der Schaft rund und/oder eckig ausgebildet. Vorzugsweise ist der Schaft vollständig oder teilweise aus Metall, Keramik oder Kunststoff ausgebildet. Die Schaftlängsachse verläuft insbesondere von dem proximalen Ende zu dem distalen Ende des Schafts. Insbesondere verläuft die Schaftlängsachse durch einen Mittelpunkt eines Querschnitts des Schafts.

Die Kameraaufnahme ist insbesondere dazu eingerichtet, in der Bildaufnahmelage das Kameramodul teilweise oder vollständig aufzunehmen. Die Kameraaufnahme kann gelenkig ausgebildet sein bzw. kann insbesondere dazu eingerichtet sein, eine Bewegung zwischen der Kameraaufnahme und dem Gliederelement freizugeben. In anderen Worten ist die Kameraaufnahme bezüglich des Gliederelements auslenkbar beweglich.

Das Kameramodul und/oder die Kameraaufnahme und/oder das wenigstens eine Gliederelement können einen ähnlichen Außendurchmesser aufweisen. Insbesondere weist das Kameramodul und/oder die Kameraaufnahme und/oder das wenigstens eine Gliederelement einen Außendurchmesser auf, der bündig mit dem Innendurchmesser des Schafts abschließt und/oder der derart ausgestaltet ist, dass das Kameramodul und/oder die Kameraaufnahme und/oder das wenigstens eine Gliederelement in den Schaft eingefügt werden können. Das Kameramodul und/oder die Kammeraufnahme können aber auch einen Außendurchmesser aufweisen, der größer als der Innendurchmesser des Schafts ist. Insbesondere kann der Außendurchmesser der Kameraaufnahme und/oder des Kameramoduls einen ähnlichen und/oder vergleichbaren und/oder identischen Außendurchmesser wie der Schaft aufweisen.

Insbesondere kann zur Realisierung der insbesondere gelenkigen Bewegbarkeit zwischen der Kameraaufnahme und dem wenigstens einen Gliederelement und/oder den Gliederelementen, eine Verbindung zwischen der Kameraaufnahme und dem Gliederelement und/oder eine Verbindung zwischen den Gliederelementen einen Schlitz aufweisen. Ansonsten kann die Verbindung zwischen der Kameraaufnahme und dem Gliederelement insbesondere einen konstanten Querschnitt aufweisen. Alternativ und/oder zusätzlich können die Kameraaufnahme und das Gliederelement und/oder die Gliederelemente über ein Gelenk, beispielsweise ein Drehgelenk und/oder ein Festkörpergelenk, verbunden sein. Die Gliederelemente können fest oder auf lösbare Weise miteinander verbunden sein.

Insbesondere kann durch die insbesondere gelenkige Bewegbarkeit zwischen der Kameraaufnahme und dem Gliederelement die Bildaufnahmelage bezüglich der Schaftlängsachse in einem ersten Winkel ausgelenkt werden. Durch die insbesondere gelenkige Bewegbarkeit zwischen zwei Gliederelementen kann insbesondere die Bildaufnahmelage bezüglich der Schaftlängsachse in einem zweiten Winkel ausgelenkt werden. D. h., wenn die insbesondere gelenkige Bewegbarkeit der Kameraaufnahme freigegeben ist bzw. die insbesondere gelenkige Bewegbarkeit zwischen der Kameraaufnahme und dem Gliederelement freigegeben ist, mit welchem die Kameraaufnahme verbunden ist, entspricht der Winkel der Bildaufnahmelage bezüglich der Schaftlängsachse dem ersten Winkel. Wenn zusätzlich die insbesondere gelenkige Bewegbarkeit des Gliederelements, mit welchem die Kameraaufnahme verbunden ist, zu einem weiteren Gliederelement freigegeben ist, entspricht der Winkel der Bildaufnahmelage bezüglich der Schaftlängsachse dem zweiten Winkel. Wenn zusätzlich die insbesondere gelenkige Bewegbarkeit des weiteren Gliederelements freigegeben wird, entspricht der Winkel der Bildaufnahmelage bezüglich der Schaftlängsachse einem weiteren Winkel. Insbesondere kann der weitere Winkel und/oder der zweite Winkel ein Vielfaches des ersten Winkels betragen.

Das stereoskopische Kameramodul umfasst insbesondere die stereoskopische Bilderfassungseinheit. Die Bilderfassungseinheit ist insbesondere in dem Kameramodul aufgenommen und damit durch das Kameramodul und/oder durch ein Gehäuse des Kameramoduls geschützt. Die stereoskopische Bilderfassungseinheit ist insbesondere in das stereoskopische Kameramodul aufgenommen. Die stereoskopische Bilderfassungseinheit kann eine erste Bilderfassungseinrichtung und eine zweite Bilderfassungseinrichtung umfassen, die jeweils in einem Spektralbereich lichtempfindlich sind. Ferner kann die stereoskopische Bilderfassungseinheit eine dritte Bilderfassungseinrichtung und/oder eine vierte Bilderfassungseinrichtung umfassen, die beispielsweise in einem weiteren Spektralbereich lichtempfindlich sind. Insbesondere ist die stereoskopische Bilderfassungseinheit ein 3D-Kamerasystem. Die erste Bilderfassungseinrichtung und/oder die zweite Bilderfassungseinrichtung können insbesondere Sensoren und Prismen umfassen, die back-to-back montiert sind. Weiter können die erste Bilderfassungseinrichtung und/oder die zweite Bilderfassungseinrichtung und/oder die dritte Bilderfassungseinrichtung und/oder die vierte Bilderfassungseinrichtung Filter und/oder eine Optik umfassen.

Mittels der ersten Bilderfassungseinrichtung und der zweiten Bilderfassungseinrichtung kann beispielsweise eine Stereobilderfassung in einem ersten Spektralbereich durchgeführt werden. Mittels der dritten Bilderfassungseinrichtung und/oder der vierten Bilderfassungseinrichtung kann beispielsweise eine Stereobilderfassung in einem zweiten Spektralbereich durchgeführt werden. Dadurch kann eine Fluoreszenzstereobilderfassung mittels der dritten Bilderfassungseinrichtung und der vierten Bilderfassungseinrichtung durchgeführt werden. Um eine Fluoreszenzbildgebung zu ermöglichen, können die Bilderfassungseinrichtungen weitere Bildsensoren umfassen, die in unterschiedlichen Spektralbereichen lichtempfindlich sind. Die erste Bilderfassungseinrichtung und die zweite Bilderfassungseinrichtung können jeweils einen ersten Bildsensor umfassen, der zumindest überwiegend in dem ersten Spektralbereich, der insbesondere dem sichtbaren Licht zugeordnet ist, lichtempfindlich ist. D.h. mittels des ersten Bildsensors ist eine Bilderfassung im Wellenlängenbereich des sichtbaren Lichts durchführbar. Dies entspricht beispielsweise einer Weißlichtbilderfassung. Die dritte Bilderfassungseinrichtung und die vierte Bilderfassungseinrichtung können jeweils einen zweiten Bildsensor umfassen, der zumindest überwiegend in dem zweiten Spektralbereich, der insbesondere dem nahinfraroten Licht zugeordnet ist, lichtempfindlich ist. D.h. mittels des zweiten Bildsensors ist eine Bilderfassung im Wellenlängenbereich des nahinfraroten Lichts durchführbar.

Alternativ können die erste und zweite Bilderfassungseinrichtung sowohl im visuellen als auch im nahen Infrarot empfindlich sein und in beiden Wellenlängenbereichen Bilder, beispielsweise Fluoreszenzbilder, erfassen.

Insbesondere umfasst das stereoskopische Kameramodul und/oder die stereoskopische Bilderfassungseinheit eine Eingangsoptik, die eine optische Achse definiert. Die Blickrichtung verläuft insbesondere entlang der optischen Achse.

Um eine einfache bzw. leichtgängige Drehbarkeit der Bilderfassungseinheit zu gewährleisten, kann die Bilderfassungseinheit innerhalb des Kameramoduls drehbar gelagert sein. Beispielsweise kann aber auch die stereoskopische Bilderfassungseinheit mit dem Kameramodul zusammen drehbar gestaltet sein. Das Kameramodul kann in diesem Fall, insbesondere in der Bildaufnahmelage an der Kameraaufnahme drehbar gelagert sein.

Insbesondere ist die stereoskopische Bilderfassungseinheit unabhängig von der Bildaufnahmelage drehbar. Damit ist es möglich, die Bildaufrichtung durch Drehung der stereoskopischen Bilderfassungseinheit von der Bildaufnahmelage und/oder dem Schrägblick des Endoskops zu entkoppeln. Insbesondere ist die Bilderfassungseinheit unabhängig davon drehbar, in welchem Winkel das Kameramodul und/oder die Bilderfassungseinheit zu der Schaftlängsachse ausgelenkt ist.

Zusätzlich kann insbesondere in der Bildaufnahmelage wenigstens eine Gliederelementlängsachse des wenigstens einen Gliederelements zu einer Kameraaufnahmelängsachse der Kameraaufnahme winklig angeordnet sein.

Insbesondere kann zwischen der Kameraaufnahmelängsachse und der Gliederelementlängsachse des Gliederelements, mit welchem die Kameraaufnahme verbunden ist, in der Bildaufnahmelage der erste Winkel realisiert werden. Zwischen den Gliederelementlängsachsen kann insbesondere ein Winkel realisiert werden, der der Differenz zwischen dem zweiten Winkel und dem ersten Winkel entspricht.

Beispielsweise kann in der Bildaufnahmelage der Winkel zwischen den Gliederelementlängsachsen dem Winkel zwischen der Kameraaufnahmelängsachse und der Gliederelementlängsachse, des Gliederelements, mit welchem die Kameraaufnahme verbunden ist, entsprechen. Alternativ kann der Winkel zwischen den Gliederelementlängsachsen unterschiedlich zu dem Winkel zwischen der Kameraaufnahmelängsachse und der Gliederelementlängsachse, des Gliederelements, mit welchem die Kameraaufnahme verbunden ist, sein. Insbesondere ist der Winkel zwischen den Gliederelementlängsachsen vergleichbar und/oder identisch. Alternativ kann der Winkel zwischen den Gliederelementlängsachsen auch unterschiedlich sein. Beispielsweise kann der Winkel zwischen den Gliederelementen in einer proximalen Richtung ansteigen. Insbesondere kann ein Gesamtwinkel zwischen der Schaftlängsachse und der Kameraaufnahmelängsachse maximal 30° und/oder 45° und/oder 75° und/oder 90° betragen.

Zur Realisierung der Bildaufnahmelage und/oder der Blickrichtung können insbesondere mehrere Gliederelemente, die zueinander beweglich, insbesondere zueinander verschwenkbar, angeordnet sind, wobei durch ein Vorschieben der Gliederelemente relativ zu dem Schaft eine Bewegbarkeit einer Anzahl an Gliederelementen zueinander freigebbar ist, wobei die Bildaufnahmelage und/oder die Blickrichtung durch die Anzahl freigegebener Gliederelemente definierbar ist.

Zudem kann die Kameraaufnahme insbesondere eine Fase aufweisen, die an einer Kante eines proximalen Endes der Kameraaufnahme angeordnet ist. Dadurch kann insbesondere die Auslenkung bzw. die insbesondere gelenkige Bewegbarkeit der Kameraaufnahme, insbesondere zu dem Gliederelement, mit welchem die Kameraaufnahme verbunden ist, definiert sein. Alternativ und/oder zusätzlich kann das wenigstens eine Gliederelement eine Fase aufweisen, die an einer Kante eines distalen Endes des Gliederelements angeordnet ist. Die Fase an der Kante an dem distalen Ende kann insbesondere der Kameraaufnahme zugewandt sein. Insbesondere können die Fase an der Kante an dem distalen Ende des Gliederelements und die Fase an der Kante an dem proximalen Ende der Kameraaufnahme in der Bildaufnahmelage aneinander anliegen. Dadurch kann insbesondere die Auslenkung bzw. die insbesondere gelenkige Bewegbarkeit der Kameraaufnahme zu dem Gliederelement, mit welchem die Kameraaufnahme verbunden ist, definiert sein.

Alternativ oder zusätzlich kann das wenigstens eine Gliederelement eine Fase aufweisen, die an einer Kante eines proximalen Endes des Gliederelements angeordnet ist. Insbesondere kann durch die Fase an der Kante des proximalen Endes des Gliederelements und einem weiteren Gliederelement und/oder einem schub- und/oder zugfesten Betätigungselement die Auslenkung bzw. die insbesondere gelenkige Bewegbarkeit des wenigstens einen Gliederelements definiert sein. Insbesondere in der Bildaufnahmelage können die jeweiligen benachbarten Fasen der Kameraaufnahme und/oder des wenigstens einen Gliederelements, die aus dem Schaft herausgeschoben sind, aneinander anliegen.

Um eine einfache und benutzerfreundliche Realisierung der Bildaufnahmelage zu realisieren und/oder um ein möglichst großes Kameramodul einsetzen zu können, kann das Kameramodul relativ zu der Kameraaufnahme bewegbar gelagert sein. Insbesondere ist das Kameramodul relativ zu der Kameraaufnahme, translatorisch und/oder in Kombination translatorisch und rotatorisch gelagert. Insbesondere kann das Kameramodul von einer ersten Position, insbesondere der Einführlage, in eine zweite Position, insbesondere der Bildaufnahmelage, relativ zu der Kameraaufnahme bewegbar sein. Beispielsweise kann die erste Position die Einführlage des Kameramoduls sein. Zudem kann insbesondere die zweite Position die Bildaufnahmelage des Kameramoduls sein und/oder eine Position sein, die das Kameramodul bezüglich der Kameraaufnahme in der Bildaufnahmelage hat und/oder ein Abstand sein, den das Kameramodul bezüglich der Kameraaufnahme in der Bildaufnahmelage hat. In anderen Worten kann sich das Kameramodul in der zweiten Position insbesondere in einer Betrachtung parallel zu einer Kameraaufnahmelängsachse der Kameraaufnahme zumindest teilweise neben der Kameraaufnahme befinden. Zudem kann das Kameramodul insbesondere in der zweiten Position weiterhin rotatorisch bezüglich der Kameraaufnahme gelagert sein.

Weiter kann das Kameramodul und/oder die Kameraaufnahme eine Steuerfläche aufweisen, die eine Aufgleitbewegungsbahn des Kameramoduls auf die Kameraaufnahme von der ersten Position in die zweite Position definiert, um eine einfache und benutzerfreundliche Realisierung der Bildaufnahmelage zu realisieren. Die Steuerflächen aus einem Material gefertigt, sodass die Aufgleitbewegungsbahn gut gleitende Eigenschaften aufweist. Insbesondere kann die Aufgleitbewegungsbahn in Form eines Gleitlagers ausgebildet sein. Das Material, aus dem die Steuerflächen gebildet sind, und/oder das Material, das die Steuerflächen aufweisen, kann Metall, insbesondere medizinischen Edelstahl, Kunststoff oder Keramik umfassen. Das Gleitlager kann außerdem mit einer reibungsreduzierenden Beschichtung versehen sein.

Um die Bildaufnahmelage einfach einstellen zu können und um ungewünschte Verdrehungen des Kabels vermeiden zu können, kann sich das Kabel entlang der Schaftlängsachse erstrecken. Alternativ oder zusätzlich kann das Kabel insbesondere bezüglich der Schaftlängsachse außermittig angeordnet sein, sodass eine Auslenkung um ein Gliederelement durch eine Zugbeaufschlagung des Kabels herstellbar ist. Um die Bildaufnahmelage einfach einstellen zu können, kann das Kabel bezüglich des gekrümmten Abschnitts außermittig und bezogen auf den Krümmungsradius des gekrümmten Abschnitts auf einer Innenseite angeordnet sein.

Um die Bildaufnahmelage einfach einstellen zu können, kann insbesondere ein Lichtleiter entlang der Schaftlängsachse zu der Kameraaufnahme geführt sein.

Alternativ oder zusätzlich kann in der Bildaufnahmelage die Kameraaufnahme und das zumindest eine Gliederelement gemeinsam einen gekrümmten Abschnitt ausbilden. Zudem kann der Lichtleiter bezüglich des gekrümmten Abschnitts außermittig und bezogen auf einen Krümmungsradius des gekrümmten Abschnitts auf einer Außenseite angeordnet sein.

Um beispielsweise die Verschmutzung zwischen der Kameraaufnahme und dem Kameramodul zu reduzieren, kann insbesondere in der Bildaufnahmelage die Kameraaufnahme distalseitig bündig mit dem Kameramodul abschließen. Bündig bedeutet insbesondere, dass geringfügige Abweichungen zwischen dem distalen Ende des Betätigungsschafts und dem distalen Ende des Kameramoduls gestattet sind.

Zur einfachen und/oder kostengünstigen Realisierung der Bildaufrichtung, kann in der Bildaufnahmelage das Kameramodul in und/oder an der Kameraaufnahme drehbar gelagert sein. Insbesondere kann das Kameramodul an einer proximalen Seite der Kameraaufnahme drehbar gelagert sein. Alternativ oder zusätzlich kann die Bilderfassungseinheit innerhalb des Kameramoduls drehbar gelagert sein.

Zur einfachen Bedienung kann die Bilderfassungseinheit insbesondere zur Bildaufrichtung mittels eines Kabels drehbar sein. Das Kabel kann insbesondere ein Torsionskabel und/oder ein Versorgungskabel sein. Das Torsionskabel kann beispielsweise auch direkt an dem Versorgungskabel entlanggeführt werden und/oder das Versorgungskabel verstärken und/oder das Versorgungskabel ummanteln. Das Kabel kann mit einer Elektronikeinheit, insbesondere einer Kamerakontrolleinheit (CCU), verbunden sein bzw. das Kabel kann das Kameramodul mit der Elektronikeinheit verbinden.

Um drehmomentschwächere Motoren mit hohen Drehzahlen einsetzen zu können, kann zwischen einem Antrieb zum Drehen des Kabels und dem Kabel eine Übersetzung angeordnet bzw. zwischengeschaltet sein. Zur Drehung des Kabels kann beispielsweise das Kabel elektrisch angetrieben sein. Die Drehung des Kabels kann auch manuell, beispielsweise durch den Benutzer, beispielsweise durch ein Drehrad, erfolgen.

Insbesondere kann zur einfachen Realisierung der Drehbarkeit zwischen dem Kabel und dem Schaft, das Kabel drehbar an einer Elektronikeinheit gelagert sein, an der das Kabel angeschlossen ist. Weiter ist es dadurch möglich, die Abdichtung des Schafts einfacher zu gestalten. Weiter kann das Kabel auch fest mit der Elektronikeinheit verbunden sein und das Kabel zusammen mit der Elektronikeinheit drehbar sein. Dadurch können kostengünstige zusammenhängende Baugruppen in der Endoskopvorrichtung verbaut werden, die das Kabel und die Elektronikeinheit sowie ggf. das Kameramodul umfassen.

Zudem kann die Endoskopvorrichtung zumindest eine Fluidleitung aufweisen, die durch den Schaft zu einem Auslass in der Kameraaufnahme verläuft, um insbesondere mittels eines Fluids, insbesondere Luft, insbesondere Druckluft, und/oder Gas, insbesondere Sterilgas, und/oder Wasser das distale Ende der Kameraaufnahme und/oder des Kameramoduls reinigen zu können. Alternativ kann die wenigstens eine Fluidleitung auch dazu verwendet werden, ein Fluid, insbesondere Druckluft, und/oder Gas, insbesondere Sterilgas, und/oder Wasser und/oder Blut und/oder andere Körperflüssigkeiten, von dem distalen Ende der Kameraaufnahme abzusaugen.

Die Kameraaufnahme kann dazu zumindest eine Öffnung für die wenigstens eine Fluidleitung aufweisen. Weiter kann die Kameraaufnahme insbesondere zumindest eine Öffnung für den Lichtleiter aufweisen, um Beleuchtungslicht nach distal auszukoppeln. Insbesondere kann der Lichtleiter an dem distalen Ende der Kameraaufnahme mit einer lichtauskoppelnden Fläche verbunden sein. Die lichtauskoppelnde Fläche kann insbesondere eine Optik umfassen.

Zur einfachen und handlichen Realisierung der Bildaufnahmelage kann die Endoskopvorrichtung weiter ein schub- und zugfestes Betätigungselement umfassen. Das Betätigungselement kann sich von einem proximalen Ende des Schafts zu einem distalen Ende des Schafts erstrecken und an das zumindest eine Gliederelement angebunden sein. Insbesondere kann durch eine Schubbeaufschlagung des Betätigungselements das zumindest eine Gliederelement und die Kameraaufnahme relativ zu dem Schaft verschiebbar sein, um die insbesondere gelenkige Bewegbarkeit den Gliederelementen und/oder um die insbesondere gelenkige Bewegbarkeit zwischen der Kameraaufnahme und dem Gliederelement freizugeben. Insbesondere durch eine Zugbeaufschlagung des Betätigungselements kann das zumindest eine Gliederelement und die Kameraaufnahme relativ zu dem Schaft zurückziehbar sein, um die insbesondere gelenkige Bewegbarkeit der Gliederelemente und/oder der Kameraaufnahme zu sperren. Das Betätigungselement kann beispielsweise in Form eines Stabs ausgebildet sein, der sich proximal an das wenigstens eine Gliederelement oder die Gliederkette anschließt und sich durch den Schaft erstreckt.

Weiter kann zur einfachen und/oder handlichen Bedienung der Endoskopvorrichtung ein proximales Ende des Betätigungselements mit einem Griff verbunden sein.

Zum einfachen Anschließen des Lichtleiters und/oder der wenigstens einen Fluidleitung, kann zudem insbesondere der Lichtleiter und/oder die wenigstens eine Fluidleitung durch das Betätigungselement und/oder das wenigstens eine Gliederelement und/oder den Griff hindurchgeführt sein.

Um eine leichtgängige Drehung des Kabels und/oder ein leichtgängiges Verschieben des schub- und zugfesten Betätigungselements zu gewährleisten, kann der Schaft einen ersten Kanal und einen zweiten Kanal aufweisen, die sich zumindest teilweise entlang der Längsachse des Schafts erstrecken. Insbesondere kann das Kabel durch den ersten Kanal hindurchgeführt sein. Weiter kann insbesondere das schub- und zugfeste Betätigungselement und/oder das zumindest eine Gliederelement durch den zweiten Kanal hindurchgeführt sein.

Um eine einfache und effektive Abdichtung der Endoskopvorrichtung zu gewährleisten, kann die Endoskopvorrichtung insbesondere einen Abdichtungskörper aufweisen, wobei der Abdichtungskörper den Schaft an der proximalen Seite abdichtet.

Um eine langlebige und/oder noch dichtere Abdichtung zu realisieren, kann der Abdichtungskörper eine erste Kammer und/oder eine zweite Kammer aufweisen, die sich zumindest teilweise entlang einer Längsachse des Abdichtungskörpers erstrecken. Dazu kann das Kabel mittels einer Dichtung, insbesondere mittels eines O-Rings gegen die erste Kammer abdichten. Vorzugsweise ist in diesem Fall das Kabel rotierbar an der Elektronikeinheit gelagert. Alternativ oder zusätzlich kann die Elektronikeinheit, die mit dem Kabel verbunden ist, mittels einer Dichtung, insbesondere mittels eines O-Rings, gegen die erste Kammer abdichten. In diesem Fall kann das Kabel insbesondere drehfest mit der Elektronikeinheit verbunden sein. D.h. zur Realisierung der Drehung des Kabels kann in diesem Fall die Elektronikeinheit mit dem Kabel mitgedreht werden. Zudem kann mittels einer Dichtung, insbesondere mittels eines O-Rings, ein schub- und zugfestes Betätigungselement gegen die zweite Kammer abdichten. Alternativ oder zusätzlich kann das Kabel mittels einer Dichtung, insbesondere mittels eines O-Rings, gegen den Schaft abdichten.

Weiter umfasst die Erfindung ein Endoskop mit einer Endoskopvorrichtung, wie sie hierin beschrieben und definiert ist.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Figur 1: eine beispielhafte Ausführungsform einer erfindungsgemäßen Endoskopvorrichtung in einer Einführlage;
- Figur 2: die beispielhafte Ausführungsform der erfindungsgemäßen Endoskopvorrichtung, die in Figur 1 dargestellt ist, in einer Konfiguration zwischen einer Einführlage und einer Bildaufnahmelage;
- Figur 3: die beispielhafte Ausführungsform der erfindungsgemäßen Endoskopvorrichtung, die in Figur 1 und 2 dargestellt ist, in der Bildaufnahmelage;
- Figur 4: eine distale Ansicht einer beispielhaften Ausführungsform einer erfindungsgemäßen Endoskopvorrichtung;
- Figur 5: eine beispielhafte Ausführungsform einer erfindungsgemäßen Endoskopvorrichtung in einer Bildaufnahmelage;
- Figur 6: eine beispielhafte Ausführungsform einer erfindungsgemäßen Endoskopvorrichtung mit einem Schaftabdichtungskörper;
- Figur 7: eine beispielhafte Ausführungsform einer erfindungsgemäßen Endoskopvorrichtung mit einem Schaftabdichtungskörper;
- Figur 8: eine Schnittdarstellung einer beispielhaften Ausführungsform einer erfindungsgemäßen Endoskopvorrichtung mit einem Schaftabdichtungskörper;

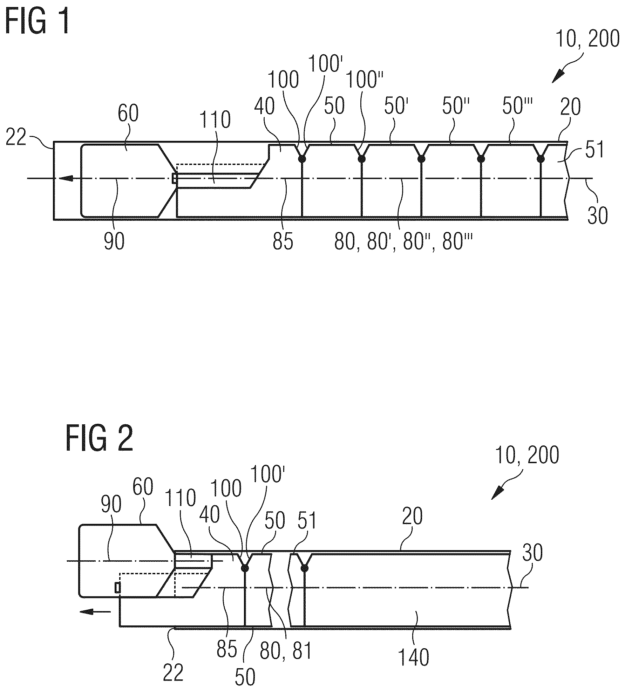

Die Figuren 1 bis 3 zeigen ein Endoskop 200 mit einer erfindungsgemäßen Endoskopvorrichtung 10. Die Endoskopvorrichtung 10 weist einen Schaft 20 mit einem distalen Ende 22, ein Kameramodul 60, eine Kameraaufnahme 40 und ein Betätigungselement 140 auf. Die Endoskopvorrichtung 10, die in den Figuren 1 und 2 gezeigt ist, weist beispielhaft mehrere Gliederelemente 50, 50', 50", 50‴ und 51 auf.

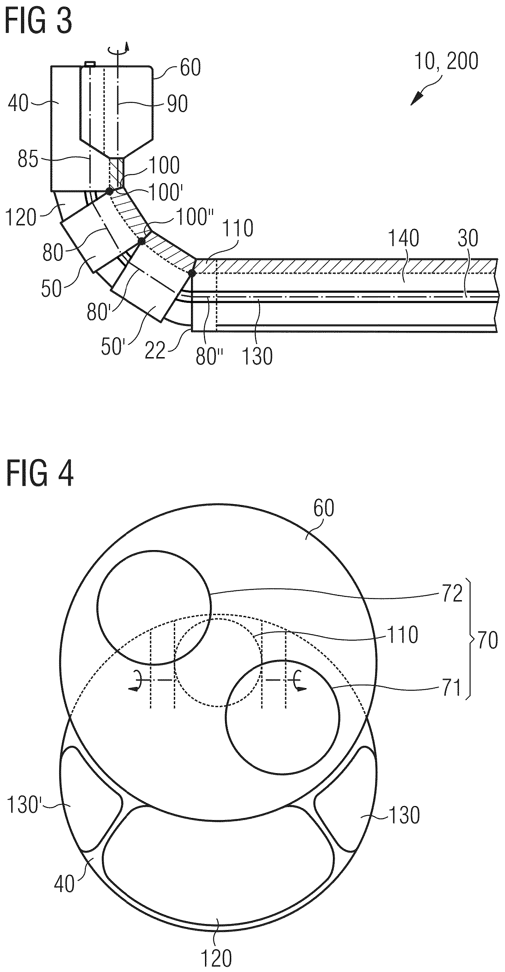

Die Endoskopvorrichtung 10, die in Figur 3 gezeigt ist, weist beispielhaft eine reduzierte Anzahl an Gliederelementen, nämlich die mehreren Gliederelemente 50 und 50' auf. Die Endoskopvorrichtung 10 kann aber auch nur ein Gliederelement 50 aufweisen. Weiter kann die Kameraaufnahme 40 auch direkt an dem Betätigungselement 140 angeordnet sein bzw. das Betätigungselement 140 kann das Gliederelement 50 bilden.

Zudem ist das Kameramodul 60 mit einem Kabel 110, insbesondere einem Torsionskabel und/oder Versorgungskabel, verbunden. Das Torsionskabel kann insbesondere um das Versorgungskabel herumgeführt sein bzw. das Torsionskabel kann das Versorgungskabel zumindest teilweise ummanteln. Das Torsionskabel kann aber auch durch das Versorgungskabel hindurchgeführt sein. Das Kameramodul 60 und/oder eine Bilderfassungseinheit, die in das Kameramodul 60 integriert ist, kann durch das Kabel 110 zur Bildaufrichtung der Bilderfassungseinheit gedreht werden. Das Kabel 110 kann insbesondere durch die Kameraaufnahme 40 und/oder die mehreren Gliederelemente 50, 50', 50", 50‴ und 51 hindurchgeführt sein. Weiter können das Kabel 110, die Kameraaufnahme 40 und/oder die mehreren Gliederelementen 50, 50', 50", 50‴ und 51 einen Schlitz aufweisen, durch den das Kabel durch den Schaft hindurchgeführt ist. Das Kabel 110 kann aber auch an der Kameraaufnahme 40 und/oder den mehreren Gliederelementen 50, 50', 50", 50‴ und/oder 51 vorbeigeführt sein. Weiter weist die Kameraaufnahme 40 an ihrem proximalen Ende eine Fase 100 auf. Weiter weisen die mehreren Gliederelemente 50, 50', 50", 50‴ und 51 jeweils an ihrem distalen Ende eine Fase 100' und an ihrem proximalen Ende eine Fase 100" auf.

Die Figur 1 zeigt eine Endoskopvorrichtung 10 in einer Einführlage. Dazu sind das Kameramodul 60, das Kabel 110, die Kameraaufnahme 40 und die mehreren Gliederelementen 50, 50', 50", 50‴ und 51 sowie das Betätigungselement 140 innerhalb des Schafts 20 angeordnet. Insbesondere das Kameramodul 60 kann in der Einführlage auch außerhalb des Schaft 20 bzw. an dem distalen Ende 22 des Schafts 20 angeordnet sein. In der Einführlage kann eine Kameralängsachse 90, eine Kameraaufnahmelängsachse 85 sowie mehrere Gliederelementlängsachsen 80, 80', 80", 80‴ und 81 parallel, insbesondere koaxial, zu einer Schaftlängsachse 30 ausgerichtet sein.

Die Figur 2 zeigt eine Endoskopvorrichtung 10 zwischen der Einführlage und einer Bildaufnahmelage bzw. eine Endoskopvorrichtung 10, die sich gerade in der Transition zwischen der Einführlage und der Bildaufnahmelage befindet. Das Kameramodul 60, das in Figur 2 dargestellt ist, ist bereits aus dem Schaft 20 herausgeschoben und die Kameraaufnahme 40, die in Figur 2 dargestellt ist, ist bereits zumindest teilweise aus dem Schaft 20 herausgeschoben. Das Kameramodul 60 wurde bereits auf die Kameraaufnahme 40 geschoben, wodurch es in radialer Richtung relativ zum Schaft 20 in eine auf der Kameraaufnahme 40 gelagerte Position verlagert wurde. Um ein leichtgängiges Verschieben des Kameramodul 60 auf die Kameraaufnahme 40 zu gewährleisten, kann das Kameramodul 60 und/oder die Kameraaufnahme 40 eine Steuerfläche aufweisen, die eine Aufgleitbewegungsbahn des Kameramoduls 60 auf die Kameraaufnahme 40 definiert.

Die Figur 3 zeigt eine Endoskopvorrichtung 10 in der Bildaufnahmelage. Insbesondere ist in der Bildaufnahmelage die Kameralängsachse 90 weiterhin parallel zur Kameraaufnahmelängsachse 85 angeordnet. Weiter ist die Kameralängsachse 90 von der Kameraaufnahmelängsachse 85 beabstandet. Die Kameraaufnahmelängsachse 85 und/oder die Kameralängsachse 90 sind jeweils in einem unterschiedlichen Winkel zu der Gliederelementlängsachse 80 des Gliederelements 50, der Gliederelementlängsachse 80' des Gliederelements 50' und der Gliederelementlängsachse 80" des Gliederelements 50" angeordnet. Das Gliederelement 50" ist weiterhin in dem Schaft 20 aufgenommen. D. h., die gelenkige Bewegbarkeit des Gliederelements 50" ist noch nicht freigegeben. Daher ist die Gliederelementlängsachse 80" des Gliederelements 50" weiterhin parallel, insbesondere koaxial, zu der Schaftlängsachse 30 des Schafts 20.

Wie dargestellt, kann in der Bildaufnahmelage die Fase 100 der Kameraaufnahme 40 auf die Fase 100' des Gliederelements 50 treffen. Alternativ oder zusätzlich kann die Fase 100" des Gliederelement 50, wie dargestellt, auf eine entsprechende Fase des Gliederelements 50' treffen. In der Endoskopvorrichtung 10, die in Figur 3 dargestellt ist, ist beispielhaft die gelenkige Bewegbarkeit der Kameraaufnahme 40, des Gliederelements 50 und des Gliederelements 50' freigegeben. Beispielhaft wird durch die freigegebene gelenkige Bewegbarkeit der Kameraaufnahme 40, des Gliederelements 50 und des Gliederelements 50' in der Bildaufnahmelage eine Blickrichtung definiert, die 90° zu der Schaftlängsachse 30 des Schafts 20 orientiert ist. D. h., in der in Figur 3 dargestellten beispielhaften Ausführungsform, wird durch die gelenkige Bewegbarkeit der Kameraaufnahme 40 und der mehreren Gliederelemente 50, 50' und 50" jeweils eine Verdrehung der Blickrichtung zu der Schaftlängsachse 30 des Schafts 20 um 30° realisiert. Die Verdrehung der Blickrichtung zu der Schaftlängsachse 30 des Schafts 20, die durch die gelenkige Bewegbarkeit der Kameraaufnahme 40 und der mehreren Gliederelemente 50, 50' und 50" realisiert wird, kann beispielsweise auch 5°, 10°, 15° und/oder 45° betragen. Insbesondere ist die Verdrehung derart groß gewählt, dass ein Abknicken des Kabels 110 und/oder der wenigstens einen Fluidleitung 130 und/oder des Lichtleiters 120 verhindert wird.

Es ist auch möglich, dass durch die gelenkige Bewegbarkeit der Kameraaufnahme 40 und der mehreren Gliederelemente 50, 50' und 50" jeweils eine unterschiedliche Verdrehung der Blickrichtung zu der Schaftlängsachse 30 des Schafts 20 realisiert wird.

Das Kabel 110 kann beispielsweise außerhalb der Gliederelementlängsachsen 80, 80', 80" und der Kameraaufnahmelängsachse 85 angeordnet sein bzw. entlang einer Linie verlaufen, die außerhalb der Gliederelementlängsachsen 80, 80', 80" und der Kameraaufnahmelängsachse 85 verläuft. In anderen Worten verläuft das Kabel 110 außermittig bezüglich der Kameraaufnahme 40 und/oder des Gliederelements 50, 50' und/oder 50".

Weiter kann die Endoskopvorrichtung 10 einen Lichtleiter 120 aufweisen. Der Lichtleiter kann beispielsweise außerhalb der Gliederelementlängsachsen 80, 80', 80" und der Kameraaufnahmelängsachse 85 angeordnet sein bzw. entlang einer Linie verlaufen, die außerhalb der Gliederelementlängsachsen 80, 80', 80" und der Kameraaufnahmelängsachse 85 verläuft. In anderen Worten verläuft der Lichtleiter 120 außermittig bezüglich der Kameraaufnahme 40 und/oder des Gliederelements 50, 50' und/oder 50".

Insbesondere können der Lichtleiter 120 und/oder das Kabel 110 jeweils auf radial gegenüberliegenden Seiten der Kameraaufnahme 40 und/oder des Gliederelements 50, 50' und/oder 50" angeordnet sein. Beispielsweise wird das Kabel 110 in der Bildaufnahmelage entlang eines Innenradius der Kameraaufnahme 40 und/oder der Gliederelemente 50, 50' und/oder 50" gebogen. Der Lichtleiter 120 kann beispielsweise entlang eines Außenradius der Kameraaufnahme 40 und/oder der Gliederelemente 50, 50' und/oder 50" gebogen sein. Dadurch, dass der Lichtleiter 120 entlang des Außenradius der Kameraaufnahme 40 und/oder der Gliederelemente 50, 50' und/oder 50" gebogen ist, wird der Lichtleiter 120 entlang eines geringeren Radius gebogen, als wenn der Lichtleiter 120 entlang des Innenradius der Kameraaufnahme 40 und/oder der Gliederelemente 50, 50' und/oder 50" gebogen ist. Dadurch kann die Lebensdauer des Lichtleiters 120 vergrößert werden. Zudem kann dadurch Beleuchtungslicht, das über den Lichtleiter 120 an dem distalen Ende der Kameraaufnahme 40, insbesondere einer lichtauskoppelnden Fläche der Kameraaufnahme 40, ausgekoppelt wird, erhöht werden.

Figur 4 zeigt eine distale Ansicht einer beispielhaften Ausführungsform einer erfindungsgemäßen Endoskopvorrichtung mit einer beispielhaften distalen Ansicht der Kameraaufnahme 40 und einer beispielhaften distalen Ansicht eines Kameramoduls 60. Das Kameramodul 60 ist mit dem Kabel 110 verbunden. Das Kabel 110 ist in der distalen Ansicht durch das Kameramodul verdeckt und daher gestrichelt gezeichnet. Das Kameramodul 60 weist eine Bilderfassungseinheit 70 mit einer ersten Bilderfassungseinrichtung 71 und einer zweiten Bilderfassungseinrichtung 72 auf, die in einem Stereobasisabstand voneinander beabstandet sind. Die Kameraaufnahme weist jeweils eine Öffnung für die Fluidleitung 130', eine Öffnung für die Fluidleitung 130 sowie die lichtauskoppelnde Fläche für den Lichtleiter 120 auf. Die Öffnungen für die Fluidleitungen 130 und/oder 130' können in Form von Düsen ausgestaltet sein. Insbesondere kann die lichtauskoppelnde Fläche der Fluidleitung 120 zwischen den Öffnungen für die Fluidleitungen 130 und 130' angeordnet sein.

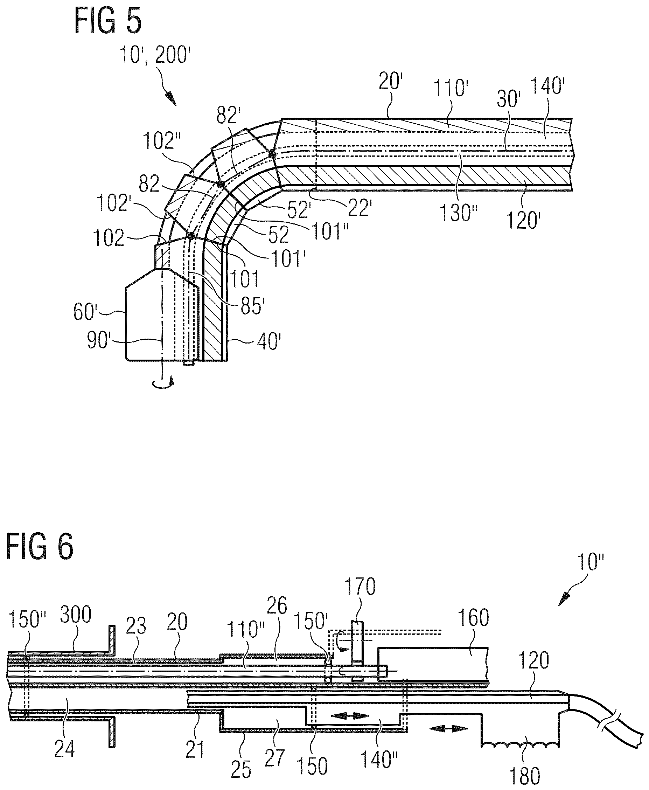

Figur 5 zeigt eine weitere beispielhafte Ausführungsform einer Endoskopvorrichtung 10' eines Endoskops 200'. Die Endoskopvorrichtung 10' weist einen Schaft 20' mit einem distalen Ende 22', ein Kameramodul 60', eine Kameraaufnahme 40', ein Betätigungselement 140 und mehrere Gliederelemente 52 und 52' auf. Weiter weist die Endoskopvorrichtung 10' ein Kabel 110', einen Lichtleiter 120' sowie wenigstens eine Fluidleitung 130" auf. Das Kabel 110' ist mit dem Kameramodul 60' verbunden. Insbesondere ist das Kabel 110' vergleichbar zu den Kabeln 110 ausgestaltet, die in den Figuren 1 bis 3 gezeigt sind. Die wenigstens eine Fluidleitung 130" sowie der Lichtleiter treten beispielsweise derart aus dem distalen Ende der Kameraaufnahme aus, wie es in Figur 4 für die Fluidleitungen 130 und 130' sowie den Lichtleiter 120 gezeigt ist.

Die Kameraaufnahme 40' und die Gliederelemente 52 und 52' weisen im Unterschied zu der Kameraaufnahme 40 und den Gliederelementen 50 und 50', die in Figur 3 gezeigt sind, unterschiedliche Fasen auf. Die Kameraaufnahme 40' weist an einem proximalen Ende die Fasen 101 und 102 auf, die an gegenüberliegenden Kanten der Kameraaufnahme 40', insbesondere an gegenüberliegenden Seiten einer Kameraaufnahmelängsachse 85', angeordnet sind. Zudem weist das Gliederelement 52 an einem distalen Ende die Fasen 101' und 102'und an einem proximalen Ende die Fasen 101" und 102" auf, die jeweils auf gegenüberliegenden Kanten des Gliederelements 52, insbesondere an gegenüberliegenden Seiten einer Gliederelementlängsachse 82, angeordnet sind. Das Gliederelement 52' weist vergleichbar zum Gliederelement 52 ebenfalls an einem distalen Ende und an einem proximalen Ende jeweils zwei Fasen an gegenüberliegenden Kanten, insbesondere an gegenüberliegenden Seiten einer Gliederelementlängsachse 82', auf.

Dadurch ist es beispielsweise möglich, dass die Endoskopvorrichtung 10' in Richtung der Kante der Kameraaufnahme 40', die die Fase 101 aufweist, und in Richtung der Kanten des Gliederelements 52, die die Fasen 101' und 101" aufweisen, gedreht wird. Insbesondere liegt in diesem Fall, insbesondere, sofern die gelenkige Bewegbarkeit der Kameraaufnahme 40' freigeben ist bzw. sofern die Kameraaufnahme 40' aus dem Schaft herausgeschoben wurde, die Kante 101 an der Kante 101' an. Zur Realisierung der Biegung kann beispielsweise das Kabel 110' in den Schaft 20' hineingeschoben und/oder der Lichtleiter 120' aus dem Schaft 20' herausgezogen werden.

Weiter kann die Endoskopvorrichtung 10' beispielsweise auch in Richtung der Kante der Kameraaufnahme 40', die die Fase 102 aufweist, und in Richtung der Kanten des Gliederelements 52, die die Fasen 102' und 102" aufweisen, gedreht werden. Insbesondere liegt in diesem Fall, insbesondere, sofern die gelenkige Bewegbarkeit der Kameraaufnahme 40' freigeben ist bzw. sofern die Kameraaufnahme 40' aus dem Schaft herausgeschoben wurde, die Kante 102 an der Kante 102' an. Zur Realisierung der Biegung kann beispielsweise das Kabel 110' aus dem Schaft 20' herausgezogen und/oder der Lichtleiter 120' in den Schaft 20' hineingeschoben werden.

Figur 6 zeigt eine beispielhafte Ausführungsform einer erfindungsgemäßen Endoskopvorrichtung 10"mit einem Schaftabdichtungskörper 25, der das proximale Ende des Schafts 20 abdichtet. Der Schaftabdichtungskörper 25 kann beispielsweise an den Schaft 20 angebracht und/oder mit dem Schaft 20 zusammen gefertigt sein. Insbesondere ist eine Verbindung zwischen der Schaftabdichtungskörper 25 und dem Schaft 20 fluiddicht ausgestaltet.

Insbesondere kann der Schaft 20 einen ersten Kanal 23 und einen zweiten Kanal 24 aufweisen. Zudem kann der Schaftabdichtungskörper 25 eine erste Kammer 26 und eine zweite Kammer 27 aufweisen. Insbesondere ist die erste Kammer 26 mit dem ersten Kanal 23 und die zweite Kammer 27 mit dem zweiten Kanal 24 verbunden. Die erste Kammer 26 und/oder die zweite Kammer 27 und/oder der erste Kanal 23 und/oder der zweite Kanal 24 können rund und/oder halbrund ausgestaltet sein. Insbesondere kann die erste Kammer 26 und/oder die zweite Kammer 27 einen größeren Durchmesser als der erste Kanal 23 und/oder der zweite Kanal 24 aufweisen.

Weiter umfasst die Endoskopvorrichtung 10" ein Kabel 110", das drehbar an einer Elektronikeinheit 160 gelagert ist und das durch den ersten Kanal 23 und die erste Kammer 26 geführt ist. Das Kabel 110" kann über eine Übersetzung 170 drehbar sein. Beispielsweise kann die Übersetzung 170 mit einem Elektromotor verbunden sein. Weiter dichtet eine Dichtung 150', insbesondere ein O-Ring, das Kabel 110" zu der ersten Kammer 26 ab.

Weiter umfasst die Endoskopvorrichtung 10" ein Betätigungselement 140", das mit einem Griff 180 verbunden ist. Zudem ist das Betätigungselement 140" durch den zweiten Kanal 24 und die zweite Kammer 27 hindurchgeführt. Zusätzlich weist die Endoskopvorrichtung 10" einen Lichtleiter 120, der durch den Griff 180 und das Betätigungselement 140" hindurchgeführt ist, auf. Weiter kann durch das Betätigungselement 140"und den Griff 180 auch wenigstens eine Fluidleitung hindurchgeführt sein. Weiter dichtet das Betätigungselement 140' mittels einer Dichtung 150, insbesondere einem O-Ring, gegen die zweite Kammer 27 ab. Weiter kann ein Abschnitt des Betätigungselement 140', an dem die Dichtung 150 angeordnet ist, einen verbreiterten Querschnitt aufweisen.

Wie dargestellt, kann der Schaft 20 durch einen Trokar 300 beispielsweise in das Körperinnere eines Patienten eingeführt werden. Um den Schaft 20 gegen den Trokar 300 abzudichten, kann beispielsweise zwischen dem Trokar 300 und dem Schaft 20 eine Dichtung 150", insbesondere ein O-Ring, angeordnet sein.

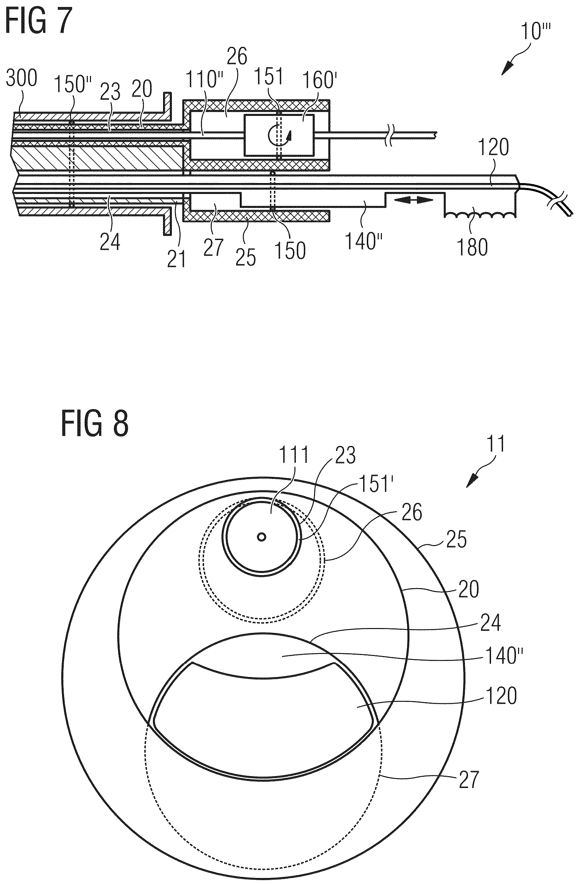

Figur 7 zeigt eine beispielhafte Ausführungsform einer erfindungsgemäßen Endoskopvorrichtung 10‴ mit dem Schaftabdichtungskörper 25, der das proximale Ende des Schafts 20 abdichtet. Im Unterschied zu der Endoskopvorrichtung, die in Figur 6 dargestellt ist, weist die beispielhafte Ausführungsform, die in Figur 7 dargestellt ist, ein Kabel 110‴ auf, das Drehfest mit einer Elektronikeinheit 160' verbunden ist. D.h. das Kabel 110‴ wird zusammen mit der Elektronikeinheit 160' gedreht. Zur Abdichtung der ersten Kammer 26 ist beispielhaft an der Elektronikeinheit 160' eine Dichtung 151, insbesondere ein O-Ring, angeordnet, der die Elektronikeinheit 160' zu der ersten Kammer 26 hin abdichtet.

Figur 8 zeigt einen beispielhaften Querschnitt einer Endoskopvorrichtung 11, in der der Schaft 20 mit dem Schaftabdichtungskörper 25 verbunden ist. Der Querschnitt verläuft durch einen Abschnitt des Schafts 20. Im Unterschied und/oder zusätzlich zu dem Kabel 110" der Endoskopvorrichtung 10", die in Figur 6 gezeigt ist, und dem Kabel 110‴ der Endoskopvorrichtung 10‴, die in Figur 7 gezeigt ist, dichtet eine Dichtung 151' ein Kabel 111 gegen den ersten Kanal 23 ab. Der Schaft 20 und der Schaftabdichtungskörper 25 weisen jeweils einen runden Querschnitt auf, deren Mittelpunkte gegeneinander verschoben bzw. voneinander beabstandet sind. Zudem weist der erste Kanal 23 und die erste Kammer 26 einen runden Querschnitt auf, wobei ein Durchmesser des ersten Kanals 23 kleiner als ein Durchmesser der ersten Kammer 26 ist. Weiter ist die zweite Kammer 27 rund ausgebildet. Eine Form des zweiten Kanals 24 wird durch die Überschneidung der zweiten Kammer 27 und dem Schaft 20 gebildet. Insbesondere weist der Lichtleiter 120 eine Rundung auf, die an die runde Form des Schafts 20 angepasst ist und wenigstens eine Rundung auf, die an die runde Form der zweiten Kammer 27 angepasst ist.

### Bezugszeichenliste

- 10, 10', 10", 10‴: Endoskopvorrichtung
- 11: Endoskopvorrichtung
- 20, 20': Schaft
- 21: proximales Ende des Schafts
- 22, 22': distales Ende des Schafts
- 23: erster Kanal
- 24: zweiter Kanal
- 25, 25': Schaftabdichtungskörper
- 26: erste Kammer
- 27: zweite Kammer
- 30, 30': Schaftlängsachse
- 40, 40': Kameraaufnahme
- 50, 50', 50", 50": Gliederelement
- 51: Gliederelement
- 52, 52': Gliederelement
- 60, 60': Kameramodul
- 70: Bilderfassungseinheit
- 71: erste Bilderfassungseinrichtung
- 72: zweite Bilderfassungseinrichtung
- 80, 80', 80", 80‴: Gliederelementlängsachse
- 81: Gliederelementlängsachse
- 82, 82': Gliederelementlängsachse
- 85, 85': Kameraaufnahmelängsachse
- 90, 90': Kameralängsachse
- 100, 100', 100": Fase
- 101, 101', 101": Fase
- 102, 102', 102": Fase
- 110, 110', 110": Kabel
- 111: Kabel
- 120, 120': Lichtleiter
- 130, 130': Fluidleitung
- 140, 140', 140": Betätigungselement
- 150, 150', 150": Dichtung
- 151, 151': Dichtung
- 160, 160': Elektronikeinheit
- 170: Übersetzung
- 180: Griff
- 200, 200': Endoskop
- 300: Trokar

## Patentansprüche

1. Endoskopvorrichtung (10), umfassend:
einen Schaft (20) mit einer Schaftlängsachse (30);
eine Kameraaufnahme (40);
wenigstens ein Gliederelement (50), an dem die Kameraaufnahme (40) beweglich befestigt ist;
ein Kameramodul (60), das eine stereoskopische Bilderfassungseinheit (70) aufweist und in der Kameraaufnahme (40) aufnehmbar ist,
wobei eine Bewegbarkeit der Kameraaufnahme (40) relativ zu dem Gliederelement (50) durch ein Verschieben der Kameraaufnahme (40) und des Gliederelements (50) relativ zu dem Schaft (20) freigebbar ist, wodurch das Kameramodul (60) von einer Einführlage in eine Bildaufnahmelage auslenkbar ist,
wobei in der Bildaufnahmelage das Kameramodul (60) eine Blickrichtung definiert, die zu der Schaftlängsachse winklig ist, und
wobei in der Bildaufnahmelage die Bilderfassungseinheit (70) zu einer Bildaufrichtung relativ zu der Kameraaufnahme (40) drehbar gelagert ist.

2. Endoskopvorrichtung (10) nach Anspruch 1,
wobei die Bilderfassungseinheit (70) unabhängig von der Bildaufnahmelage drehbar ist, insbesondere ist die Bilderfassungseinheit (70) unabhängig davon drehbar, in welchem Winkel das Kameramodul (60) und/oder die Bilderfassungseinheit (70) zu der Schaftlängsachse (30) ausgelenkt ist.

3. Endoskopvorrichtung (10) nach Anspruch 1 oder 2,
wobei in der Bildaufnahmelage wenigstens eine Gliederelementlängsachse (80) des wenigstens einen Gliederelements (50) zu einer Kameraaufnahmelängsachse (90) der Kameraaufnahme (40) winklig angeordnet ist.

4. Endoskopvorrichtung (10) nach einem der vorherigen Ansprüche, umfassend:
mehrere Gliederelemente (50), die zueinander beweglich, insbesondere zueinander verschwenkbar, angeordnet sind, wobei durch ein Vorschieben der Gliederelemente (50) relativ zu dem Schaft (20) die Bewegbarkeit einer Anzahl an Gliederelementen (50) zueinander freigebbar ist, wobei die Bildaufnahmelage durch die Anzahl freigegebener Gliederelemente (50) definierbar ist.

5. Endoskopvorrichtung (10) nach einem der vorherigen Ansprüche,
wobei die Kameraaufnahme (40) eine Fase (100) aufweist, die an einer Kante eines proximalen Endes der Kameraaufnahme (40) angeordnet ist, und/oder
wobei das wenigstens eine Gliederelement (50) eine Fase (100') aufweist, die an einer Kante eines distalen Endes des Gliederelements (50) angeordnet ist, und/oder eine Fase (100") aufweist, die an einer Kante eines proximalen Endes des Gliederelements (50) angeordnet ist;
wobei in der Bildaufnahmelage die jeweiligen benachbarten Fasen (100, 100', 100") der Kameraaufnahme (40) und/oder des wenigstens einen Gliederelements (50), die aus dem Schaft (20) herausgeschoben sind, aneinander anliegen.

6. Endoskopvorrichtung (10) nach einem der vorherigen Ansprüche,
wobei das Kameramodul (60) relativ zu der Kameraaufnahme (40) bewegbar gelagert, insbesondere translatorisch und/oder in Kombination translatorisch und rotatorisch gelagert, ist,
wobei das Kameramodul (60) von einer ersten Position in eine zweite Position relativ zu der Kameraaufnahme (40) bewegbar ist, und
wobei sich das Kameramodul (60) in der zweiten Position in einer Betrachtung parallel zu einer Kameraaufnahmelängsachse der Kameraaufnahme (60) zumindest teilweise neben der Kameraaufnahme (40) befindet.

7. Endoskopvorrichtung (10) nach Anspruch 6,
wobei das Kameramodul (60) und/oder die Kameraaufnahme (40) eine Steuerfläche aufweist, die eine Aufgleitbewegungsbahn des Kameramoduls (60) auf die Kameraaufnahme (40) von der ersten Position in die zweite Position definiert.

8. Endoskopvorrichtung (10) nach einem der vorherigen Ansprüche, weiter umfassend:
ein Kabel (110), das an das Kameramodul (60) angeschlossen ist und sich entlang der Schaftlängsachse (30) erstreckt,
wobei das Kabel (110) bezüglich der Schaftlängsachse (30) außermittig angeordnet ist, sodass eine Auslenkung um das Gliederelement (50) durch eine Zugbeaufschlagung des Kabels (110) herstellbar ist.

9. Endoskopvorrichtung (10) nach einem der vorhergehenden Ansprüche, weiter umfassend:
einen Lichtleiter (120) der entlang der Schaftlängsachse (30) zu der Kameraaufnahme (40) geführt ist,
wobei in der Bildaufnahmelage die Kameraaufnahme (40) und das zumindest eine Gliederelement (50) gemeinsam einen gekrümmten Abschnitt ausbilden, und wobei der Lichtleiter (120) bezüglich des gekrümmten Abschnitts außermittig und bezogen auf einen Krümmungsradius des gekrümmten Abschnitts auf einer Außenseite angeordnet ist.

10. Endoskopvorrichtung (10) nach Anspruch 8 und 9,
wobei das Kabel (110) bezüglich des gekrümmten Abschnitts außermittig und bezogen auf den Krümmungsradius des gekrümmten Abschnitts auf einer Innenseite angeordnet ist

11. Endoskopvorrichtung (10) nach einem der vorherigen Ansprüche,
wobei in der Bildaufnahmelage die Kameraaufnahme (40) distalseitig bündig mit dem Kameramodul (60) abschließt.

12. Endoskopvorrichtung (10) nach einem der vorherigen Ansprüche,
wobei in der Bildaufnahmelage die Kameraaufnahme (40) das Kameramodul (60) drehbar lagert und/oder die Bilderfassungseinheit (70) innerhalb des Kameramoduls (60) drehbar gelagert ist.

13. Endoskopvorrichtung (10) nach einem der vorherigen Ansprüche,
wobei die Bilderfassungseinheit (70) zur Bildaufrichtung durch ein Kabel (110, 110'), insbesondere ein Torsionskabel und/oder ein Versorgungskabel, drehbar ist.

14. Endoskopvorrichtung (10) nach einem der vorhergehenden Ansprüche, umfassend:
ein schub- und zugfestes Betätigungselement (180), das sich von einem proximalen Ende des Schafts (21) zu einem distalen Ende des Schafts (22) erstreckt und an das zumindest eine Gliederelement (50) angebunden ist,
wobei durch eine Schubbeaufschlagung des Betätigungselements (140) das zumindest eine Gliederelement (50) und die Kameraaufnahme (40) relativ zu dem Schaft (20) verschiebbar sind, um die Bewegbarkeit zwischen den Gliederelementen (50) freizugeben und/oder um die Bewegbarkeit zwischen der Kameraaufnahme (40) und dem Gliederelement (50) freizugeben, und
wobei durch eine Zugbeaufschlagung des Betätigungselements (140) das zumindest eine Gliederelement (50) und die Kameraaufnahme (40) relativ zu dem Schaft (20) zurückziehbar sind, um die Bewegbarkeit der Gliederelemente (50) und/oder der Kameraaufnahme (40) zu sperren.

15. Endoskopvorrichtung (10) nach Anspruch 14,
wobei ein proximales Ende des Betätigungselements (180) mit einem Griff (120) verbunden ist.

16. Endoskopvorrichtung (10) nach einem der Ansprüche 14 oder 15,
wobei ein Lichtleiter (120) und/oder wenigstens eine Fluidleitung (130) durch das Betätigungselement (180) und/oder das wenigstens eine Gliederelement (50) und/oder den Griff (120) hindurchgeführt ist.

17. Endoskopvorrichtung (10) nach einem der vorhergehenden Ansprüche,
wobei der Schaft (20) einen ersten Kanal (23) und einen zweiten Kanal (24) aufweist, die sich zumindest teilweise entlang der Längsachse des Schafts (20) erstrecken,
wobei ein Kabel (110) durch den ersten Kanal (23) hindurchgeführt ist, und
wobei ein schub- und zugfestes Betätigungselement (180) und/oder das zumindest eine Gliederelement (50) durch den zweiten Kanal (24) hindurchgeführt ist.

18. Endoskopvorrichtung (10) nach einem der vorhergehenden Ansprüche, umfassend:
einen Abdichtungskörper (25),
wobei der Abdichtungskörper (25) den Schaft (20) an der proximalen Seite abdichtet.

19. Endoskopvorrichtung (10) nach Anspruch 18,
wobei der Abdichtungskörper (25) eine erste Kammer (26) und/oder eine zweite Kammer (27) aufweist, die sich zumindest teilweise entlang einer Längsachse des Abdichtungskörpers (25) erstrecken,
wobei mittels einer Dichtung, insbesondere mittels eines O-Rings, ein Kabel (110) und/oder eine Elektronikeinheit (160'), die mit dem Kabel (110') verbunden ist, gegen die erste Kammer (26) abdichtet, und/oder
wobei mittels einer Dichtung, insbesondere mittels eines O-Rings, ein schub- und zugfestes Betätigungselement (180) gegen die zweite Kammer (27) abdichtet.

20. Endoskop (200) mit einer Endoskopvorrichtung (10) nach einem der vorhergehenden Ansprüche.
